# EUROPÄISCHE PATENTSCHRIFT

(11) **EP 2 134 886 B1**
(45) Veröffentlichungstag und Bekanntmachung des Hinweises auf die Patenterteilung: **02.03.2011**
(21) Anmeldenummer: 08716484.4
(22) Anmeldetag: 12.03.2008
(51) Int. Cl.: C23C 18/08, C23C 18/12, C23C 18/14, G02B 1/12, G02B 3/00

(54) **VERFAHREN ZUR HERSTELLUNG VON FLÄCHIGEN GRÖSSEN- ODER ABSTANDSVARIATIONEN IN MUSTERN VON NANOSTRUKTUREN AUF OBERFLÄCHEN**
METHOD FOR THE CREATION OF PLANAR VARIATIONS IN SIZE OR DISTANCE IN NANOSTRUCTURE PATTERNS ON SURFACES
PROCÉDÉ DE PRODUCTION DE VARIATIONS DE TAILLES ET DE DISTANCES PLATES DE MOTIFS DE NANOSTRUCTURES SUR DES SURFACES

(30) Priorität: 11.04.2007 DE 102007017032
(43) Veröffentlichungstag der Anmeldung: 23.12.2009
(60) Teilanmeldung: 10015574.6
(73) Patentinhaber: Max-Planck-Gesellschaft zur Förderung der Wissenschaften e.V., 80539 München (DE)
(72) Erfinder: SPATZ, Joachim, 89520 Heidenheim (DE); LOHMÜLLER, Theobald, 90473 Nürnberg (DE); ARNOLD, Marco, 69251 Gaiberg (DE)
(74) Vertreter: Katzameyer, Michael
(86) Internationale Anmeldenummer: PCT/EP2008/001981
(87) Internationale Veröffentlichungsnummer: WO 2008/125172

(56) Entgegenhaltungen:
- EP-B- 1 027 157
- US-A- 5 891 366
- US-A1- 2002 145 132
- US-A1- 2005 059 760
- MULLER M ET AL: "ORDERING AND PACKING PERIODICITY OF AU- CONTAINING BLOCK COPOLYMER MICELLES" POLYMERIC MATERIALS SCIENCE AND ENGINEERING, WASHINGTON, DC, US, Bd. 90, 1. Januar 2004 (2004-01-01), Seiten 255-256, XP008101682 ISSN: 0743-0515 in der Anmeldung erwähnt
- J. P. SPATZ ET AL.: "Micellar Inorganic-Polymer Hybrid Systems - A Tool for Nanolithography" ADVANCED MATERIALS, Bd. 11, Nr. 2, 1999, Seiten 149-153, XP002513693 Weinheim
- J. C. MEINERS ET AL.: "Adsorption of Block-Copolymer Micelles from a Selective Solvent" MACROMOLECULES, Bd. 30, Nr. 17, 1997, Seiten 4945-4951, XP002513694 Washington DC
- G. KÄSTLE ET AL.: "Micellar Nanoreactors - Preparation and Characterization of Hexagonally Ordered Arrays of Metallic Nanodots" ADVANCED FUNCTIONAL MATERIALS, Bd. 13, Nr. 11, November 2003 (2003-11), Seiten 853-861, XP002513695 Weinheim
- L. ZHOU ET AL: "Scanning thermal microscopy and atomic force microscopy studies of laer-induced deposited metal lines" APPLIED SURFACE SCIENCE, Bd. 120, 1997, Seiten 149-158, XP002523305
- D. POONDI ET AL: "Synthesis of silver nanoparticles by a laser-liquid-solid interaction technique" JOURNAL OF MATERAILS SYNTHESIS AND PROCESSING, Bd. 6, Nr. 2, 1998, Seiten 89-104, XP002523306

## Beschreibung

Die Darstellung von chemischen und topographischen Variationen auf der Nano- und Mikrometerskala sind unter anderem für Anwendungen auf den Gebieten der refraktiven und diffraktiven Optik, der Selektion molekular wichtiger Längenskalen bei der Zelladhäsion und der Einzelmolekülanbindung an Grenzflächen von großem technischen Interesse. Dies gilt insbesondere für graduelle Veränderungen der Topographie und chemischen Eigenschaften einer Oberfläche.

Die Erzeugung von molekular wohl definierten Gradienten bietet z.B. die Möglichkeit, richtungsgesteuerte Prozesse wie die Zellbewegung, die externe chemische Gradienten erfordert, zu untersuchen. Besonders auf dem Gebiet der zellulären Haptotaxie sind chemische Gradienten mit molekularer Präzision wünschenswert, um biologische Reaktionen qualitativ und quantitativ detailliert zu untersuchen. Efimenko und Genzer ((Adv. Mater. 13, 2001), 1560-1563) haben gezeigt, dass eine Feinstellung der Pfropfdichte von Molekülen auf Oberflächen durch die Herstellung mechanisch assemblierter Monoschichten (MAMs) möglich ist. MAMS sind Strukturen, welche durch eine Kombination von Selbstassemblierung, mechanischer Streckung eines PDMS-Trägers und molekularer Diffusion hergestellt werden. Das Verfahren zur Herstellung dieser MAMs ist durch die Inkorporation eines mechanischen Schrittes relativ aufwendig und darüber hinaus nur für flexible Träger und nicht für starre Substrate, wie z.B. Glas, Metall, Siliciumverbindungen etc., geeignet. Einen weiteren Nachteil stellt die Tatsache dar, dass eine Molekül-Molekül-Abstandsregulierung durch mechanisches Strecken eines Substrats nicht möglich ist, da das Clustern von einzelnen Molekülen nicht wesentlich eingeschränkt werden kann.

EP 1 027 157 B1, M. Möller et al. in Polym. Mat. Sci. Eng. 90, 2555-256 (2004), J.C. Meiners et al. in Macromolecules 30, 4945-4951 (1997), J.P. Spatz et al. in Advanced Materials, Bd. 11, Nr. 2, 149-153 (1999), und G. Kästle et al. in Advanced Functional Materials, Bd. 13, Nr. 11, 853-861 (2003), beschreiben jeweils Verfahren zur Herstellung von Nanostrukturen auf einem Substrat unter Verwendung von Mizellen eines organischen Blockcopolymers ohne auf die Problematik der Erzeugung von gezielten Abstandsvariationen in diesen Nanostrukturen einzugehen.

Vor diesem Hintergrund besteht die Aufgabe der vorliegenden Erfindung in der Bereitstellung eines verbesserten, einfachen und vielseitig anwendbaren Verfahrens mit dem flächige Größen- und/oder Abstandsvariationen in Mustern von Nanostrukturen auf Oberflächen so präzise eingestellt werden können, dass definierte Gradientenoberflächen erhalten werden.

Diese Aufgabe wurde erfindungsgemäß gelöst durch eine Weiterentwicklung der mizellaren Nanolithographie (siehe z.B. EP 1 027 157). Bei der mizellaren Nanolithographie wird eine mizellare Lösung eines Blockcopolymers auf ein Substrat abgeschieden, z.B. durch Tauchbeschichtung, und bildet unter geeigneten Bedingungen auf der Oberfläche eine geordnete Filmstruktur von chemisch unterschiedlichen Polymerdomänen, die unter anderem von Typ, Molekulargewicht und Konzentration des Blockcopolymers abhängt. Die Mizellen in der Lösung können mit anorganischen Salzen beladen werden, die nach der Abscheidung mit dem Polymerfilm zu anorganischen Nanopartikeln reduziert werden können. Es wurde nun erfindungsgemäß festgestellt, dass sowohl die laterale Separationslänge der genannten Polymerdomänen und damit auch der resultierenden Nanopartikel als auch die Größe dieser Nanopartikel durch verschiedene Maßnahmen so präzise flächig eingestellt werden können, dass nanostrukturierte Oberflächen mit gewünschten Abstands-und/oder Größengradienten herstellbar sind.

Gegenstand der vorliegenden Erfindung sind somit Verfahren zur flächigen Größen oder Abstandsvariation in Mustern von Nanostrukturen auf einem Substrat gemäß dem unabhängigen Anspruch 1. Spezielle oder bevorzugte Ausführungsfor-men der Erfindung sind Gegenstand der abhängigen Ansprüche.

### Beschreibung der Erfindung

Wie bereits oben erwähnt, beruht die vorliegende Erfindung auf einer Weiterentwicklung der Technik der mizellaren Nanolithographie (siehe z.B. EP 1 027 157). Bei der mizellaren Nanolithographie wird eine mizellare Lösung eines Diblock-oder Multiblockcopolymers auf ein Substrat abgeschieden, z.B. durch Tauchbeschichtung, und bildet unter geeigneten Bedingungen auf der Oberfläche eine geordnete Filmstruktur von chemisch unterschiedlichen Polymerdomänen, die unter anderem von Typ, Molekulargewicht und Konzentration des Blockcopolmers abhängt. Beispielsweise sind die Abstände der einzelnen Polymerdomänen voneinander abhängig vom Molekulargewicht und der Konzentration des Blockcopolymers in der Lösung. Die Mizellen in der Lösung können mit anorganischen Salzen bzw. Säuren beladen werden, die nach der Abscheidung mit dem Polymerfilm zu anorganischen Nanopartikeln reduziert werden können.

Es wurde nun erfindungsgemäß festgestellt, dass sowohl die laterale Separationslänge der genannten Polymerdomänen und damit auch der resultierenden Nanopartikel als auch die Größe dieser Nanopartikel durch verschiedene Maßnahmen so präzise flächig eingestellt werden können, dass nanostrukturierte Oberflächen mit gewünschten Abstands- und/oder Größengradienten herstellbar sind.

Einen wesentlichen Schritt bei der Entwicklung der vorliegenden Erfindung stellte die Erkenntnis dar, dass durch die Variation der Ziehgeschwindigkeit mit der das zu beschichtende Substrat aus einer mizellaren Lösung gezogen wird, eine Feineinstellung der lateralen Separationslänge der abgeschiedenen Polymerdomänen bzw. der resultierenden Nanopartikel für die Herstellung von Gradientenoberflächen ermöglicht wird.

Von Möller et al., (Polym. Mat., Science und Engineering, 2004, 90, 255) war zwar bereits festgestellt worden, dass sowohl der Ordnungsgrad als auch die Abstände von Nanostrukturen, die mittels der mizellarer Nanolithographie erzeugt worden waren, von der (konstanten) Ziehgeschwindigkeit abhingen, mit der ein Glimmer-Substrat aus der mizellaren Lösung gezogen wurde. In Anbetracht der beobachteten relativ starken Veränderung des Ordnungsgrades mit der Ziehgeschwindigkeit unter einigen untersuchten Bedingungen lassen diese ersten Versuchsergebnisse jedoch die Herstellung eines Separationslängengradienten mit der nötigen Präzision und dem nötigen Ordnungsgrad eher unrealistisch erscheinen und wurde dort auch nicht erörtert oder vorgeschlagen. Insbesondere wurde hier auch eine Molekulargewichtsverteilung zwischen den Blöcken PS und P2VP eingesetzt, die für eine kontrollierte Veränderung des Abstands zwischen Mizellen ungeeignet ist. Wünschenswert ist hier ein Mw (PS) >> Mw (P2VP).

Die Auswertung von hier beschriebenen umfangreichen Versuchen mit verschiedenen mizellaren Lösungen, aus denen gleiche Substrate mit unterschiedlichen, jedoch konstanten Ziehgeschwindigkeiten gezogen wurden (siehe Beispiel 2), ergab zwei wichtige Ergebnisse. Erstens, dass für alle getesteten Lösungen ein Bereich nahezu linearer Abnahme der Distanzen mit höheren Ziehgeschwindigkeiten erhalten werden konnte, und zweitens, dass der Ordnungsgrad der Nanostrukturen zwar mit der Ziehgeschwindigkeit variierte, jedoch durch Wahl geeigneter Polymere und Polymerkonzentrationen trotzdem hoch gehalten werden konnte. Dies führte zu der Schlussfolgerung, dass es möglich sein sollte, durch graduelle Variation der Ziehgeschwindigkeit, mit der eine Substratoberfläche aus einer mizellaren Lösung gezogen wird, auch einen Gradienten der lateralen Separationslänge der Polymerdomänen bzw. Nanopartikel auf der Substratoberfläche zu erzeugen.

Demgemäß umfasst das erfindungsgemäße Verfahren zur flächigen Größen- oder Abstandsvariation in Mustern von Nanostrukturen auf einem Substrat in einer Ausführungsform
a) Kontaktieren eines Substrats mit einer flüssigen Phase, die mit einer anorganischen Metallverbindung (z.B. einem Metallsalz) beladene Mizellen eines organischen Zweiblock- oder Multiblockcopolymers enthält, durch Eintauchen in diese flüssige Phase, wobei auf dem Substrat chemisch unterschiedliche Polymerdomänen, die in Mizellen eingeschlossene anorganische Metallverbindungen (z.B. Metallsalze) umfassen, abgeschieden werden;
b) Herausziehen des Substrats aus der flüssigen Phase mit einer vorbestimmten Ziehgeschwindigkeit, wobei die Ziehgeschwindigkeit kontinuierlich oder stufenweise variiert wird, so dass ein Gradient der lateralen Separationslänge der Polymerdomänen auf der Substratoberfläche erzeugt wird; und
c) Überführung der anorganischen Metallverbindungen in den abgeschiedenen Polymerdomänen durch eine Oxidations- oder Reduktionsbehandlung in anorganische Nanopartikel und gegebenenfalls vollständige oder teilweise Entfernung des organischen Polymers durch eine Plasmabehandlung, wobei die Positionen und laterale Separationslänge der erhaltenen Nanopartikel von den Positionen und der lateralen Separationslänge der abgeschiedenen Polymerdomänen bestimmt werden.

Typischerweise wird die Ziehgeschwindigkeit in Schritt b) in einem Bereich von 0,1 mm/min bis 100 mm/min, vorzugsweise von 1 mm/min bis 20 mm/min, kontinuierlich oder stufenweise variiert.

Das Verfahren kann beispielsweise so durchgeführt werden, dass auf eine Phase relativ geringer kontinuierlicher Geschwindigkeitsänderung eine abrupte stufenweise Geschwindigkeitsänderung folgt. Auf diese Weise können z.B. nacheinander mehrere verschiedene Separationslängen-Gradienten auf der gleichen Substratoberfläche erzeugt werden.

Als Zweiblock- oder Multiblockcopolymer in diesem Verfahren kann grundsätzlich jedes Mizellen-bildende Blockcopolymer verwendet werden, welches als Film auf ein Substrat abgeschieden werden kann und dabei eine geordnete Struktur verschiedener Polymerdomänen bildet. Geeignete Blockcopolymere sind beispielsweise alle in der oben zitierten EP 1 027 157 genannten Blockcopolymere. In einer spezielleren Ausführungsform ist das Zweiblock- oder Multiblockcopolymer aus der Gruppe aus Polystyrol(n)-b-Poly(2-vinylpyridin(m), Polystyrol(n)-b-Poly(4-vinylpyridin(m), Polystyrol(n)-b-Poly(ethylenoxid)(m), wobei n und m die Anzahl der Wiederholungseinheiten angeben und unabhängig voneinander ganze Zahlen im Bereich von 10-10000, insbesondere 100-1000, sind, ausgewählt. Vorzugsweise wird das Molekulargewicht (Mw) (gelöster Block) >> Mw (schlecht gelöster Block) gewählt.

Als anorganische Verbindungen, mit denen die Mizellen in der Lösung bzw. die Polymerdomänen in dem abgeschiedenen Kunststofffilm beladen werden können, sind grundsätzlich alle anorganischen Metallverbindungen (z.B. Metallsalze) geeignet, die durch Oxidation oder Reduktion in anorganische Nanopartikel überführt werden können. Geeignete Salze sind beispielweise alle in der oben zitierten EP 1 027 157 genannten Metallsalze. Vorzugsweise umfassen die erfindungsgemäß verwendeten Metallsalze mindestens ein Salz der Metalle Au, Pt, Pd, Ag, In, Fe, Zr, Al, Co, Ni, Ga, Sn, Zn, Ti, Si oder Ge. Besonders bevorzugt ist HAuCl₄.

In einer speziellen Ausführungsform des erfindungsgemäßen Verfahrens geschieht die reduktive Überführung der Metallverbindungen in den auf der Substratoberfläche abgeschiedenen Polymerdomänen in Nanopartikel in Schritt c) durch Bestrahlung mit einem lateral entlang der Substratoberfläche intensitätsmodulierten Lichtfeld, wodurch abhängig von der Lichtintensität an verschiedenen Positionen der Substratoberfläche unterschiedlich große Nanopartikel erzeugt werden.

Nach oder gleichzeitig mit der Überführung der Metallverbindungen in anorganische Nanopartikel kann das organische Polymer ganz oder teilweise durch eine entsprechende Behandlung entfernt werden. Diese Behandlung ist typischerweise eine Plasmabehandlung, z.B. mit einem Sauerstoff- oder Wasserstoffplasma, wie im Stand der Technik bekannt. Nähere Angaben zu einer solchen Behandlung sind z.B. ebenfalls in der EP 1 027 157 zu finden.

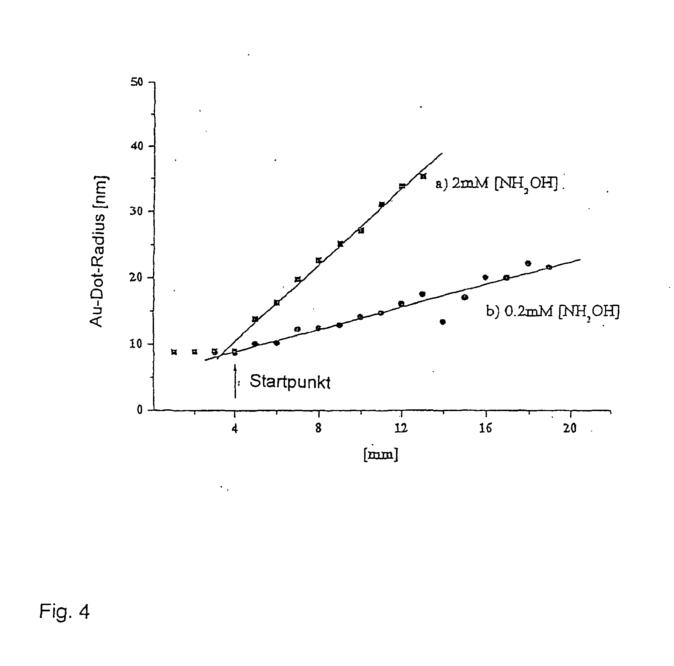

In einer spezielleren Ausführungsform des erfindungsgemäßen Verfahren umfasst es ferner die folgenden Schritte:
d) Kontaktieren des Substrats mit den nach Schritt c) erhaltenen Nanopartikeln mit einer flüssigen Phase, die eine Lösung einer anorganischen Metallverbindung enthält (z.B. HAuCl₄ in 0,01 - 10 mM NH₂OH), durch Eintauchen in diese flüssige Phase, wobei gelöstes Material auf den Nanopartikeln aufwächst und diese damit vergrößert; und
e) Herausziehen des Substrats aus der flüssigen Phase nach einer vorbestimmten Zeitspanne, wodurch eine gewünschte Größe der Nanopartikel eingestellt wird (siehe z.B. Fig. 4).

Hierbei kann durch eine Variation der Ziehgeschwindigkeit in Schritt e) auch ein Gradient der Partikelgröße auf der Substratoberfläche erzeugt werden. Die Partikelgröße kann dabei in einem Bereich von etwa 1-200 nm auf einer Länge > 100 µm eingestellt werden.

Typischerweise wird die Ziehgeschwindigkeit in Schritt e) in einem Bereich von 0,1 mm/min bis 100 mm/min, vorzugsweise von 1 mm/min bis 20 mm/min, kontinuierlich oder stufenweise variiert.

In einer spezielleren Ausführungsform dieses Verfahrens werden die Nanopartikel auf der Substratoberfläche während der Schritte d) und e) mit einem lateral entlang der Substratoberfläche intensitätsmodulierten Lichtfeld bestrahlt, wodurch aufgrund der unterschiedlichen Wachstumsgeschwindigkeit der Nanopartikel, welche abhängig von der Lichtintensität ist, an verschiedenen Positionen der Substratoberfläche unterschiedlich große Nanopartikel erzeugt werden. Die Wellenlänge beträgt etwa 120-500 nm, die Lichtstärke kann je nach Bedarf über einen breiten Bereich von sehr klein bis sehr groß variieren.

Auch durch die Bestrahlung mit einem intensitätsmodulierten Lichtfeld kann ein Gradient der Partikelgröße erzeugt werden. Die Partikelgröße kann dabei in einem Bereich von etwa 1-200 nm auf einer Länge > 0,3 µm fein eingestellt werden.

In einer bevorzugten Ausführungsform des erfindungsgemäßen Verfahrens werden mehrere der oben beschriebenen Maßnahmen gleichzeitig oder nacheinander durchgeführt, so dass auf einer nanostrukturierten Substratoberfläche sowohl mindestens ein Separationslängengradient als auch mindestens ein Partikelgrößengradient der Nanopartikel erzeugt wird.

Bei den obigen Verfahrensvarianten, in denen eine Bestrahlung mit intensitätsmoduliertem Licht vorgenommen wird, kann die Intensität des Lichtfeldes flächig graduell oder stufenweise durch Interferenz und Beugung an statischen oder dynamisch veränderbaren optischen Elementen eingestellt werden. Eine typische Vorrichtung hierfür umfasst z.B. folgende Komponenten: Lichtquelle, Spiegel, Interferenzgitter, diffraktive optische Elemente. Letztere können im Falle von Flüssigkristallen oder Mikrospiegeln musteraktiv geschaltet werden und so hinsichtlich ihrer Interferenzeigenschaften aktiv (dynamisch) beeinflusst werden.

### Kurzbeschreibung der Figuren

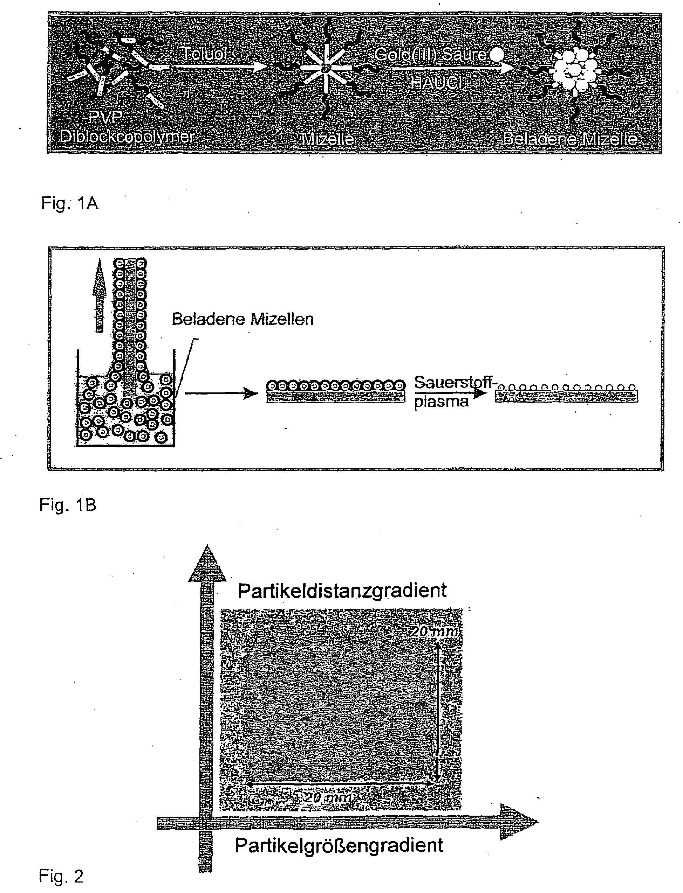

**Fig. 1A** zeigt schematisch die Bildung einer Mizelle aus einem PS-PVP-Diblockcopolymer und die Beladung mit einer Gold(III)-Verbindung.
**Fig. 1B** zeigt schematisch die Aufbringung beladener Mizellen auf ein Substrat und die spätere Entfernung des Polymerfilms durch eine Plasmabehandlung.
**Fig. 2** zeigt eine Photographie eines zweifachen Gradienten aus Gold-Nanopartikeln.

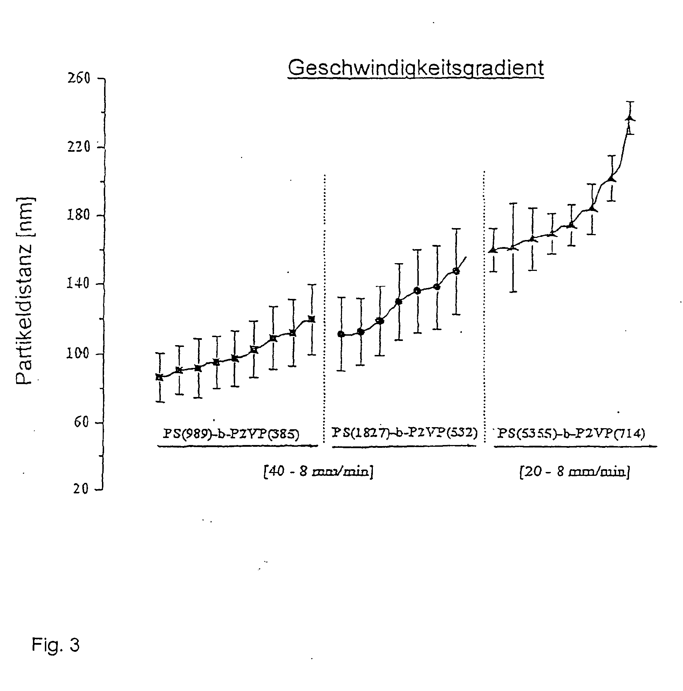

**Fig. 3** zeigt die Variation des Partikelabstands als Funktion der Ziehgeschwindigkeit eines Substrats aus einer mizellaren Lösung.
**Fig. 4** zeigt die Variation der Partikelgröße als Funktion der Position auf einem Substrat.
**Fig. 5 A-D** zeigen die mittleren Au-Goldpartikel-Separationslängen, die bei verschiedenen Ziehgeschwindigkeiten für verschiedene Copolymerlösungen A-D erhalten wurden.
**Fig. 6** zeigt die Korrelation zwischen dem Ordnungsparameter, den Interpartikeldifferenzen und den. Ziehgeschwindigkeiten für diese Lösungen A-D.
**Fig. 7** zeigt repräsentative rasterelektronenmikroskopische Aufnahmen der Nanomuster, die von verschiedenen Separationslängengradienten bei einer Position am Anfang des Gradienten, als das Substrat mit hoher Ziehgeschwindigkeit aus der entsprechenden Blockcopolymerlösung herausgezogen wurde (A1, B1, C1), und einer Position am Ende des Gradienten mit einer niedrigen Ziehgeschwindigkeit (A2, B2, C2) erhalten wurden.
**Fig. 8** zeigt die entlang der Gradienten gemessenen Interpartikeldistanzen (Fig 8A-C) und die Gestaltung der Gradienten (Fig. 8D).
**Fig. 9** gibt die Interpartikeldistanzen von zwei konvers orientierten Gradienten auf einem Substrat als Plot gegen die Eintauchtiefe an und zeigt rasterelektronenmikroskopische Aufnahmen von verschieden Positionen auf jedem Substrat.

Die folgenden Beispiele dienen zur näheren Erläuterung der vorliegenden Erfindung, ohne diese jedoch darauf zu beschränken.

### BEISPIEL 1

### Abstandsvariation von Gold-Nanopartikeln durch die Verwendung verschiedener konstanter Geschwindigkeiten, um das Substrat aus einer mizellaren Lösung zu ziehen

Es wurden verschiedene Lösungen A, B, C und D hergestellt, deren Charakteristiken in der folgenden Tabelle 1 zusammenfasst sind.

**Tabelle 1**

| Mizellare Lösung | Polymer | L = HauCl₄/P2VP | Polymer- Konz. (mg/ml) |
|---|---|---|---|
| A | PS(1780)-b- P2VP(520) | 0,2 | 1 |
| B | PS(1780)-b- P2VP(520) | 0,2 | 2 |
| C | PS(990)-b- P2VP(385) | 0, 3 | 3 |
| D | PS(990)-b- P2VP(385) | 0, 3 | 5 |

Diese Lösungen wurden hergestellt durch Lösen des jeweiligen Blockcopolymers in der vorgesehenen Konzentration in trockenem Toluol und anschließendes Zugeben von HAuCl₄ x H₂O im angegebenen Verhältnis L, wobei die Mischung solange gerührt wurde, bis die Tetrachlorgoldsäure vollständig solubilisiert war.

Für jede Lösung wurden 10 Proben (Glas-Objektträger) sorgfältig gereinigt, z.B. mit "Piranha"-Lösung (H₂O₂/H₂SO₄ = 1:1), mit reinem Wasser getrocknet, in einem Stickstoffstrom getrocknet und in die jeweilige mizellare Lösung getaucht und mit verschiedenen Geschwindigkeiten herausgezogen. Nach einer Wasserstoff-plasmabehandlung (z.b. wie in EP 1 027 157 beschrieben) wurden die Gold-Nanopartikel-Distanzen mit dem Rasterelektronenmikroskop bestimmt. Hierfür wurden 5 verschiedene Bereiche auf jedem Glasträger (20 x 20 mm) bei einer Vergrößerung von 5000 analysiert.

Zur Berechnung der jeweiligen mittleren Interpartikeldistanzen und der Standardabweichungen wurde das Computer-Analyseprogramm Origin 7.0 eingesetzt. Die 5 erhaltenen Nanopartikel-Separationswerte mit ihren jeweiligen Standardabweichungen wurden gemittelt, um die mittleren Gesamt-Interpartikeldistanzen für einen einzelnen Objektträger, der mit einer bestimmten Ziehgeschwindigkeit aus der Lösung des Diblockcopolymers herausgezogen wurde, für jede der 10 verschiedenen Ziehgeschwindigkeiten zu ermitteln.

**Tabelle 2**

| Mizellare Lösung | Minimale Interpartikeldistanz (nm) ± Standardabweichung | Minimale Interpartikeldistanz (nm) ± Standardabweichung | Δ (nm) |
|---|---|---|---|
| A | 128 ± 18 | 175 ± 28 | 47 |
| B | 93 ± 13 | 125 ± 18 | 32 |
| C | 58 ± 9 | 93 ± 14 | 35 |
| D | 59 ± 9 | 94, ± 14 | 35 |

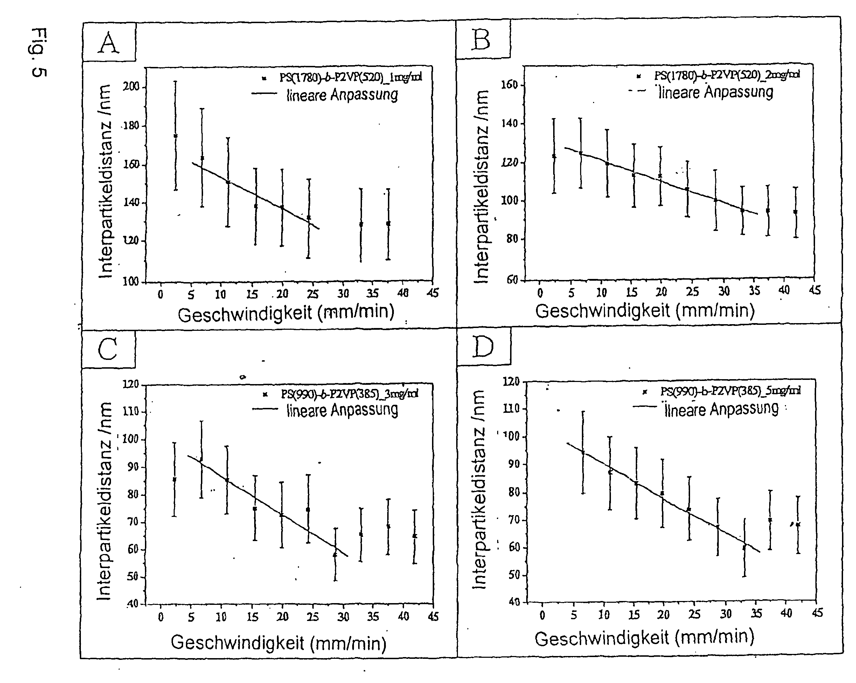

Fig. 5A-D zeigt alle getesteten Lösungen und die jeweiligen korrespondierenden Interpartikeldistanzen bei jeder Ziehgeschwindigkeit. Diese Studie demonstrierte, dass in allen getesteten Lösungen eine nahezu lineare Abnahme der Distanzen mit höheren Ziehgeschwindigkeiten erhalten werden konnte. Der maximale Unterschied in der lateralen Beabstandung der Gold-Nanopartikel für die getesteten Lösungen war etwa 40 nm. Es ist bemerkenswert, dass bei einer bestimmten Geschwindigkeit die Beabstandung der Gold-Nanopartikel eine Sättigung zu erfahren scheint. Anderseits wurde eine Abnahme der Musterordnung bei einer sehr geringen Geschwindigkeit (2,3 mm/min) beobachtet, die zu stark differierenden Interpartikeldistanzen an verschiedenen Messpositionen auf dem Substrat führte.

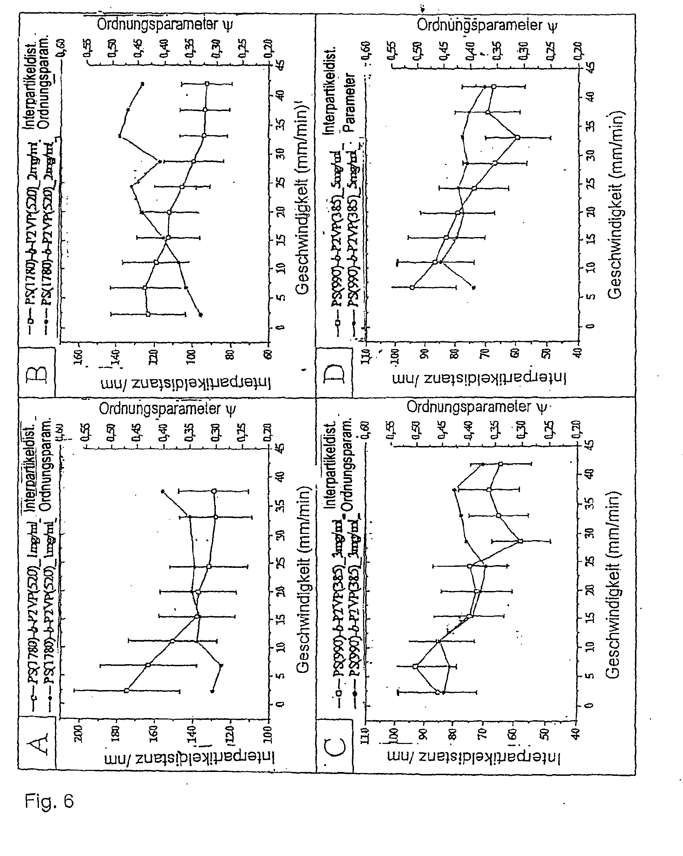

Fig. 6 zeigt die Korrelation zwischen dem Ordnungsparameter, den Interpartikeldifferenzen und den Ziehgeschwindigkeiten. Dabei zeigten die Lösungen A und B eine Erhöhung des Ordnungsparameters mit zunehmender Geschwindigkeit, während der Ordnungsparameter konstant blieb oder leicht abnahm, wenn die Ziehgeschwindigkeit in den Lösungen C und D erhöht wurde. Offenbar führt eine zu niedrige Mizellendichte auf der Oberfläche (z.B. aufgrund einer zu niedrigen Polymerkonzentration oder einer zu niedrigen Ziehgeschwindigkeit) zu einem geringeren Ordnungsgrad auf der Oberfläche und es kann gefolgert werden, dass es für jedes Diblockcopolymer eine optimale Mizellendichte in einer monomizellaren Schicht bezüglich der Musterqualität gibt.

### BEISPIEL 2

### Separationslängengradienten von Gold-Nanopartikeln durch Variation der Ziehgeschwindigkeit des Substrats aus einer mizellaren Lösung

Bei diesem Experiment wurden drei Proben mit Gradienten von 1 mm, 2 mm und 3 mm Länge hergestellt. Die Variation der Ziehgeschwindigkeit aus der mizellaren Lösung, hergestellt analog zu Beispiel 1, variierte von 40 mm/min ("Ausgangsgeschwindigkeit") bis 8 mm/min ("Endgeschwindigkeit").

Die Substrate wurden mit einem Elektromotor bewegt, dessen Ziehgeschwindigkeit über die programmierbare Spannung seiner Energieversorgung eingestellt werden konnte.

**Tabelle 3**

| | Polymer | L = HauCl₄/P2VP | Polymer- Konz. (mg/ml) | Gradientenlänge (mm) | Steigung (nm/mm) |
|---|---|---|---|---|---|
| Gradient 1 | PS(990)-*b*- P2VP(385) | 0,3 | 3 | 1 | 21 ± 9 |
| Gradient 2 | PS(990)-*b*- P2VP(385) | 0,3 | 3 | 2 | 10 ± 6 |
| Gradient 3 | PS(990)-*b*- P2VP(385) | 0, 3 | 5 | 3 | 7 ± 4 |

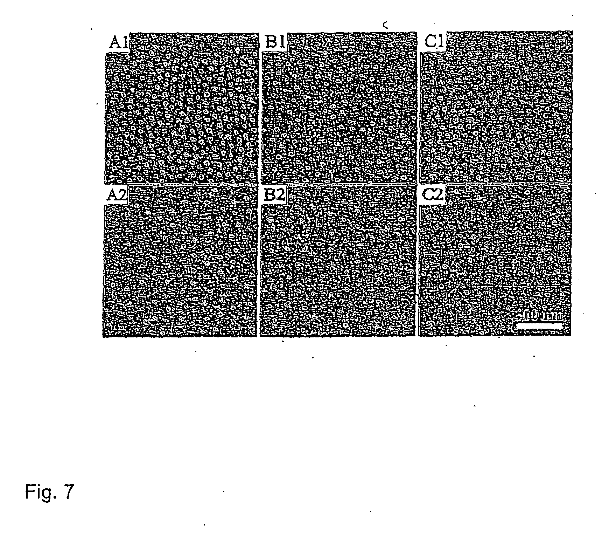

Fig. 7 zeigt rasterelektronenmikroskopische Aufnahmen der Nanomuster, die von jedem Gradientensubstrat bei einer Position am Anfang des Gradienten, als das Substrat mit 40 mm/min Ziehgeschwindigkeit aus der entsprechenden Blockcopolymerlösung herausgezogen wurde (A1, B1, C1), und einer Position am Ende des Gradienten mit einer Ziehgeschwindigkeit von 8 mm/min (A2, B2, C2) erhalten wurden (A Gradient 1; B Gradient 2; C Gradient 3). Die Rasterelektronenmikroskop-Aufnahmen demonstrieren, dass keine Beeinträchtigung der Gold-Nanomuster festzustellen ist, wenn ein einziges Substrat mit den angegebenen verschiedenen Geschwindigkeiten herausgezogen wird.

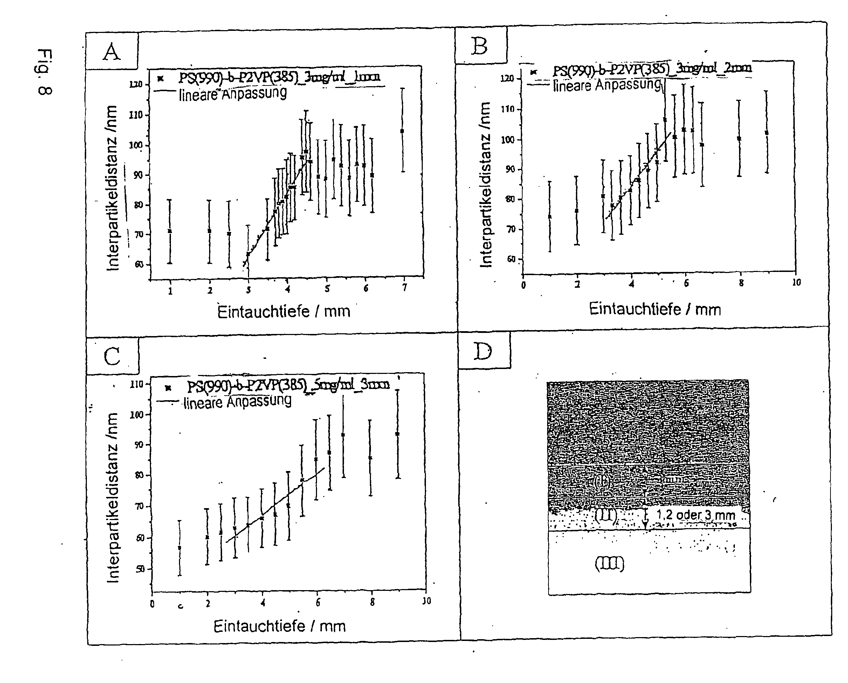

Fig. 8 zeigt die entlang der Gradienten gemessenen Interpartikeldistanzen (Fig 8A-C) und die Gestaltung der Gradienten (Fig. 8D). Aus Fig. 8A-C ist ersichtlich, dass eine nahezu lineare Zunahme der Interpartikeldistanten auftrat, welche der linearen Abnahme der Ziehgeschwindigkeit zuzuschreiben ist. Dies bedeutet, dass für kürzere Gradientenlängen steilere Steigungen erhalten wurden. Die Steigung eines Gradienten ist durch das Verhältnis der Gradientenlänge zur Zunahme der Interpartikeldistanzen angegeben. Alle Gradienten begannen und endeten an Positionen, welche durch ein Computerprogramm vorgegeben wurden.

### BEISPIEL 3

### Herstellung von mehreren Separationslängengradienten auf einem Substrat

In diesem Experiment wurden zwei verschiedene Gradienten auf einem Substrat erzeugt. Dazu wurde die Ziehgeschwindigkeit für den ersten Gradienten von 8 auf 40 mm/min erhöht und für den zweiten Gradienten von 40 auf 8 mm/min verringert.

**Tabelle 4**

| Eigenschaften zweier konvers orientierter Gradienten | | | | | | |
|---|---|---|---|---|---|---|
| | Polymer | L = HauCl₄/P2VP | Polymer- Konz. (mg/ml) | Gradientenlänge (mm) | Steigungen (nm/mm) | |
| | | | | | 1 | 2 |
| Gradient 4 | PS(1780)-*b*- P2VP (520) | 0,2 | 2 | 3 | - 11 ± 5 | + 11 ± 5 |
| Gradient 5 | PS(990)-*b*- P2VP(385) | 0,3 | 3 | 1 | -31 ±8 | 23 ± 9 |

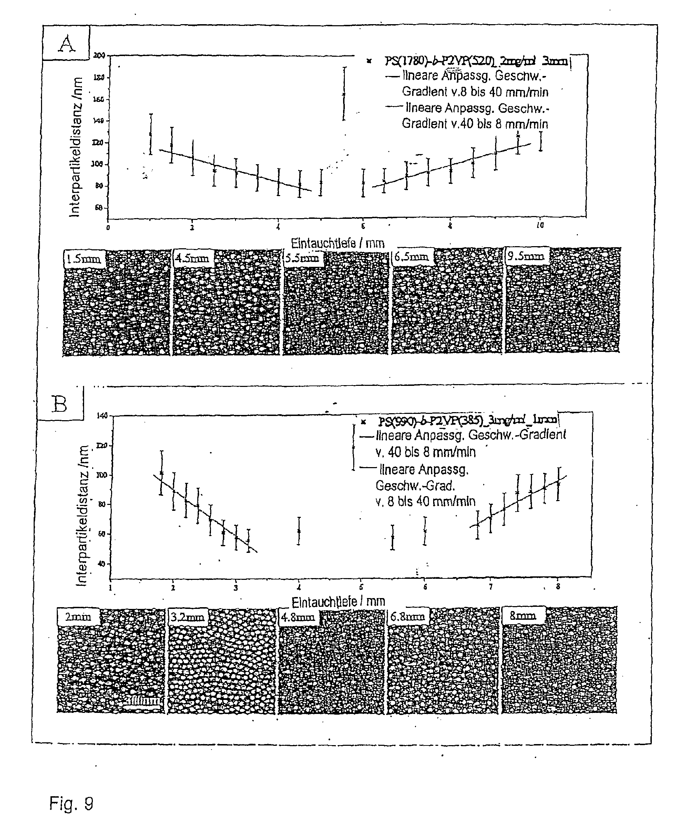

Fig. 9 gibt die Interpartikeldistanzen dieser konvers orientierten Gradienten als Plot gegen die Eintauchtiefe (entspricht der Gradientenlänge) an und zeigt rasterelektronenmikroskopische Aufnahmen von verschiedenen Positionen auf jedem Substrat.

Die Bestimmung der Interpartikeldstanzen ergab Resultate, die mit denjenigen von Einzelgradienten vergleichbar waren. Diese Ergebnisse belegen, dass durch Änderung der Ziehgeschwindigkeit eine Gradientenbildung in beiden Richtungen möglich war. Die ungewöhnlich hohen Interpartikeldistanzen, die in der Mitte des Substrats beobachtet wurden, können durch den plötzlichen Stopp auf 0/min von einer Geschwindigkeit von 40 mm/min erklärt werden und können als Bereiche betrachtet werden, die mit sehr geringer Geschwindigkeit herausgezogen wurden.

### BEISPIEL 4

### Partikelgrößengradienten von Gold-Nanopartikeln durch Variation der Ziehgeschwindigkeit

Durch Variation der Ziehgeschwindigkeit von 0,01 bis 100 mm/min eines bereits nanostrukturierten Glasplättchens aus Lösungen folgender Zusammensetzung: a) wässriges 2 mM NH₂O + 1 mg/mL HAuCl₄, b) wässriges 0,2 mM NH₂OH + 1 mg/mL HAuCl₄ wurde ein relativ steiler Partikelgradient von 10-40 nm auf einer Strecke von etwa 13 mm (2 mM [NH₂O]) bzw. ein flacherer Partikelgradient von etwa 10-25 nm (0,2 mM [NH₂OH]) auf einer Strecke von etwa 15 mm erzeugt (siehe Fig. 4).

### BEISPIEL 5

### Überlagerung eines Partikelgradienten mit einem dazu senkrechten Separationslängengradienten

In Anlehnung an Beispiel 2 wird zuerst ein Separationslängengradient auf einem Substrat erzeugt, dann das Substrat um 90° gedreht und analog zu Beispiel 4 ein Partikelgradient erzeugt.

## Patentansprüche

1. Verfahren zur flächigen Größen- oder Abstandsvariation in Mustern von Nanostrukturen auf einem Substrat, umfassend
a) Kontaktieren eines Substrats mit einer flüssigen Phase, die mit einer anorganischen Metallverbindung beladene Mizellen eines organischen Zweiblock- oder Multiblockcopolymers enthält, durch Eintauchen in diese flüssige Phase, wobei auf dem Substrat chemisch unterschiedliche Polymerdomänen, die in Mizellen eingeschlossene Metallverbindungen umfassen, abgeschieden werden;
b) Herausziehen des Substrats aus der flüssigen Phase mit einer vorbestimmten Ziehgeschwindigkeit, wobei die Ziehgeschwindigkeit kontinuierlich oder stufenweise variiert wird, so dass ein Gradient der lateralen Separationslänge der Polymerdomänen auf der Substratoberfläche erzeugt wird;
c) Überführung der Metallverbindungen in den abgeschiedenen Polymerdomänen durch eine Oxidations-oder Reduktionsbehandlung in anorganische Nanopartikel und gegebenenfalls vollständige oder teilweise Entfernung des organischen Polymers durch eine Plasmabehandlung, wobei die Positionen und laterale Separationslänge der erhaltenen Nanopartikel von den Positionen und der lateralen Separationslänge der abgeschiedenen Polymerdomänen bestimmt werden.

2. Verfahren nach Anspruch 1, **dadurch gekennzeichnet**, das die Ziehgeschwindigkeit in Schritt b) in einem Bereich von 0,1 mm/min bis 100 mm/min, vorzugsweise von 1 mm/min bis 20 mm/min, variiert wird.

3. Verfahren nach Anspruch 2, **dadurch gekennzeichnet, dass** nacheinander mehrere verschiedene Separationslängen-Gradienten auf der gleichen Substratoberfläche erzeugt werden.

4. Verfahren nach einem der Ansprüche 1-3, **dadurch gekennzeichnet, dass** die reduktive Überführung der Metallverbindungen in den auf der Substratoberfläche abgeschiedenen Polymerdomänen in Nanopartikel in Schritt c) durch Bestrahlung mit einem lateral entlang der Substratoberfläche intensitätsmodulierten Lichtfeld geschieht, wodurch abhängig von der Lichtintensität an verschiedenen Positionen der Substratoberfläche unterschiedlich große Nanopartikel erzeugt werden.

5. Verfahren nach einem der Ansprüche 1-4, **dadurch gekennzeichnet, dass** es ferner die folgenden Schritte umfasst:
d) Kontaktieren des Substrats mit den nach Schritt c) erhaltenen Nanopartikeln mit einer flüssigen Phase, die eine Lösung einer anorganischen Metallverbindung enthält, durch Eintauchen in diese flüssige Phase, wobei gelöstes Material auf den Nanopartikeln aufwächst und diese damit vergrößert; und
e) Herausziehen des Substrats aus der flüssigen Phase nach einer vorbestimmten Zeitspanne, wodurch eine gewünschte Größe der Nanopartikel eingestellt wird.

6. Verfahren nach Anspruch 5, **dadurch gekennzeichnet, dass** durch Variation der Ziehgeschwindigkeit in Schritt e) ein Gradient der Partikelgröße auf der Substratoberfläche erzeugt wird.

7. Verfahren nach Anspruch 4 oder 5, **dadurch gekennzeichnet, dass** die Nanopartikel auf der Substratoberfläche während der Schritte d) und e) mit einem lateral entlang der Substratoberfläche intensitätsmodulierten Lichtfeld bestrahlt werden, wodurch aufgrund der unterschiedlichen Wachstumsgeschwindigkeit der Nanopartikel, welche abhängig von der Lichtintensität ist, an verschiedenen Positionen der Substratoberfläche unterschiedlich große Nanopartikel erzeugt werden.

8. Verfahren nach Anspruch 4 oder 7, **dadurch gekennzeichnet, dass** durch die Bestrahlung mit einem intensitätsmodulierten Lichtfeld ein Gradient der Partikelgröße erzeugt wird.

9. Verfahren nach Anspruch 6 oder 8, **dadurch gekennzeichnet, dass** auf einer nanostrukturierten Substratoberfläche sowohl mindestens ein Separationslängengradient als auch mindestens ein Partikelgrößengradient der Nanopartikel erzeugt wird.

10. Verfahren nach einem der Ansprüche 1-9, **dadurch gekennzeichnet, dass** die anorganische Metallver-bindung mindestens ein Salz der Metalle Au, Pt, Pd, Ag, In, Fe, Zr, Al, Co, Ni, Ga, Sn, Zn, Ti, Si oder Ge umfasst.

11. Verfahren nach einem der Ansprüche 1-10, **dadurch gekennzeichnet, dass** das Zweiblock- oder Multiblockcopolymer aus der Gruppe aus Polystyrol(n)-b-Poly(2-vinylpyridin(m), Polystyrol(n)-b-Poly(4-vinylpyridin(m), Polystyrol(n)-b-Poly(ethylenoxid) (m), wobei n und m die Anzahl der Wiederholungseinheiten angeben und unabhängig voneinander ganze Zahlen im Bereich von 10-10000, insbesondere 100-1000, sind, ausgewählt sind.

12. Verfahren nach einem der Ansprüche 4 oder 7, **dadurch gekennzeichnet, dass** die Intensität des Lichtfeldes flächig graduell oder stufenweise durch Interferenz und Beugung an statischen oder dynamisch veränderbaren optischen Elementen eingestellt wird.

## Claims

1. A method for the extensive variation of sizes or distances in patterns of nanostructures on a substrate, comprising
a) contacting a substrate with a liquid phase that contains micelles of an organic two-block- or multi-block copolymer, which micelles are charged with an inorganic metal compound, by immersion into this liquid phase, during which chemically different polymer domains comprising metal compounds enclosed in micelles are deposited on the substrate;
b) withdrawing of the substrate from the liquid phase at a predetermined withdrawing speed, which withdrawing speed is varied continuously or gradually, so that a gradient of the lateral separation length of the polymer domains is produced on the substrate surface;
c) conversion of the metal compounds in the deposited polymer domains by an oxidation- or reduction treatment into inorganic nanoparticles and optionally complete or partial removal of the organic polymer by a plasma treatment, wherein the positions and lateral separation length of the nanoparticles obtained are determined by the positions and the lateral separation length of the deposited polymer domains.

2. The method according to Claim 1, **characterized in that** the withdrawing speed in step b) is varied in a range of 0.1 mm/min to 100 mm/min, preferably 1 mm/min to 20 mm/min.

3. The method according to Claim 2, **characterized in that** several different separation length gradients are produced in series on the same substrate surface.

4. The method according to one of Claims 1-3, **characterized in that** the reductive conversion of the metal compounds in the polymer domains deposited on the substrate surface into nanoparticles takes place in step c) by irradiation with a light field intensity-modulated laterally along the substrate surface, as a result of which differently sized nanoparticles are produced as a function of the light intensity at different positions of the substrate surface.

5. The method according to one of Claims 1-4, **characterized in that** the method further comprises the following steps:
d) contacting of the substrate with the nanoparticles obtained according to step c) with a liquid phase containing a solution of an inorganic metal compound by immersion into this liquid phase, during which dissolved material grows on the nanoparticles and thus enlarges them; and
e) withdrawing the substrate from the liquid phase after a predetermined time period, as a result of which a desired size of the nanoparticles is adjusted.

6. The method according to Claim 5, **characterized in that** a gradient of the particle size is produced on the substrate surface by variation of the withdrawing speed in step e).

7. The method according to Claim 4 or 5, **characterized in that** the nanoparticles are irradiated on the substrate surface during the steps d) and e) with a light field intensity-modulated laterally along the substrate surface, as a result of which differently sized nanoparticles are produced at different positions of the substrate surface on account of the differing growth speed of the nanoparticles that is a function of the light intensity:

8. The method according to Claim 4 or 7, **characterized in that** a gradient of the particle size is produced by the irradiation with an intensity-modulated light field.

9. The method according to Claim 6 or 8, **characterized in that** at least one separation length gradient as well as at least one particle size gradient of the nanoparticles is produced on a nanostructured substrate surface.

10. The method according to one of Claims 1-9, **characterized in that** the inorganic metal compound comprises at least one salt of the metals Au, Pt, Pd, Ag, In, Fe, Zr, Al, Co, Ni, Ga, Sn, Zn, Ti, Si or Ge.

11. The method according to one of Claims 1-10, **characterized in that** the two-block- or multi-block copolymer is selected from the group of polystyrene (n)-b-poly (2-vinylpyridine (m), polystyrene (n)-b-poly (4-vinylpyridine (m), polystyrene (n)-b-poly (ethylene oxide) (m), in which n and m indicate the number of repetition units and are, independently of one another, integers in the range of 10-10,000, in particular 100-1000.

12. The method according to one of Claims 4 or 7, **characterized in that** the intensity of the light field is adjusted gradually or in stages in an extensive area by interference and diffraction on static or dynamically variable optical elements.

## Revendications

1. Procédé de production de variations de dimensions ou de distances planes dans des motifs de nanostructures sur un substrat, comprenant les étapes consistant à :
a) mettre un substrat en contact avec une phase liquide qui contient des micelles d'un copolymère à blocs de deux ou plusieurs espèces monomères chargées d'un composé métallique inorganique en le plongeant dans cette phase liquide afin d'y déposer ainsi des domaines polymères chimiquement différents contenant des composés métalliques inclus dans des micelles ;
b) retirer le substrat de la phase liquide à une vitesse de retrait prédéterminée, ladite vitesse de retrait variant en continu ou par paliers de façon à générer un gradient de longueur de séparation latérale des domaines polymères sur la surface du substrat ;
c) convertir par un traitement d'oxydation ou de réduction les composés métalliques contenus dans les domaines polymères déposés en nanoparticules inorganiques et le cas échéant éliminer complètement ou partiellement le polymère organique par un traitement au plasma, les positions et la longueur de séparation latérale des nanoparticules obtenues étant définies par les positions et les longueurs de séparation latérale des domaines polymères déposés.

2. Procédé selon la revendication 1, **caractérisé en ce que** la vitesse de retrait à l'étape b) varie dans une plage allant de 0,1 mm/min à 100 mm/min, de préférence de 1 mm/min à 20 mm/min.

3. Procédé selon la revendication 2, **caractérisé en ce que** plusieurs gradients de longueur de séparation différents sont générés successivement sur la même surface du substrat.

4. Procédé selon l'une des revendications 1 à 3, **caractérisé en ce que** la conversion réductrice des composés métalliques contenus dans les domaines polymères déposés sur la surface du substrat en nanoparticules à l'étape c) se fait par exposition au rayonnement d'un champ lumineux modulé en intensité latéralement le long de la surface du substrat, des nanoparticules de tailles différentes étant produites dans différentes positions de la surface du substrat en fonction de l'intensité lumineuse.

5. Procédé selon l'une des revendications 1 à 4, **caractérisé en ce qu'**il comprend en outre les étapes suivantes consistant à :
d) mettre le substrat en contact avec les nanoparticules obtenues après l'étape c) en utilisant une phase liquide qui contient une solution d'un composé métallique inorganique en le plongeant dans cette phase liquide, le matériau dissous s'accumulant sur les nanoparticules et augmentant ainsi leur taille ; et
e) retirer le substrat de la phase liquide après une durée prédéterminée afin d'ajuster ainsi la taille voulue pour les nanoparticules.

6. Procédé selon la revendication 5, **caractérisé en ce que** la variation de la vitesse de retrait à l'étape e) permet de générer un gradient de taille de particules sur la surface du substrat.

7. Procédé selon la revendication 4 ou la revendication 5, **caractérisé en ce que** les nanoparticules présentes sur la surface du substrat sont exposées pendant les étapes d) et e) au rayonnement d'un champ lumineux modulé en intensité latéralement le long de la surface du substrat, les vitesses de croissance différentes des nanoparticules, qui dépendent de l'intensité lumineuse, dans différentes positions à la surface du substrat donnant des nanoparticules de tailles différentes.

8. Procédé selon la revendication 4 ou la revendication 7, **caractérisé en ce que** l'exposition à un champ lumineux modulé en intensité permet de générer un gradient de tailles de particules.

9. Procédé selon la revendication 6 ou la revendication 8, **caractérisé en ce qu'**au moins un gradient de longueur de séparation ainsi qu'au moins un gradient de tailles des nanoparticules est généré sur une surface nanostructurée du substrat.

10. Procédé selon l'une des revendications 1 à 9, **caractérisé en ce que** le composé métallique inorganique comprend au moins un sel des métaux Au, Pt, Pd, Ag, In, Fe, Zr, Al, Co, Ni, Ga, Sn, Zn, Ti, Si ou Ge.

11. Procédé selon l'une des revendications 1 à 10, **caractérisé en ce que** le copolymère à blocs de deux ou plusieurs espèces monomères est choisi dans le groupe constitué par les polystyrène(n)-b-poly(2-vinylpyridine(m), polystyrène(n)-b-poly-(4-vinylpyridine)(m), polystyrène(n)-b-polyoxyde d'éthylène(m), où n et m indiquent le nombre des motifs répétitifs et sont indépendamment l'un de l'autre des nombres entiers valant de 10 à 10 000, en particulier de 100 à 1 000.

12. Procédé selon l'une des revendications 4 ou 7, **caractérisé en ce que** l'intensité du champ lumineux est ajustée dans le plan graduellement ou par paliers par interférence et diffraction sur des éléments optiques modifiables de manière statique ou dynamique.
